# EUROPEAN PATENT SPECIFICATION

(11) **EP 1 572 227 B1**
(45) Date of publication and mention of the grant of the patent: **15.02.2012**
(21) Application number: 03799485.2
(22) Date of filing: 16.12.2003
(51) Int. Cl.: A61K 38/17, A61K 38/43, A61K 38/45, A61K 38/46, A61P 3/00

(54) **PIK4CB INVOLVED IN THE REGULATION OF ENERGY HOMEOSTASIS**
PIK4CB INVOLVIERT IN DER REGULIERUNG DES ENERGIESTOFFWECHSELS
PIK4CB IMPLIQUEE DANS L'HOMEOSTASE ENERGETIQUE

(30) Priority: 16.12.2002 EP 02028275; 20.12.2002 EP 02028609; 30.12.2002 EP 02029081
(43) Date of publication of application: 14.09.2005
(73) Proprietor: DeveloGen Aktiengesellschaft, 37079 Göttingen (DE)
(72) Inventor: MEISE, Martin, 58675 Hemer (DE); EULENBERG, Karsten, 79639 Grenzach/Wyhlen (DE); MOLITOR, Andreas, 37077 Göttingen (DE); STEUERNAGEL, Arnd, 37085 Göttingen (DE); NGUYEN, Tri, 37081 Göttingen (DE); KATTERLE, Yvonne, 14482 Potsdam (DE)
(74) Representative: Weiss, Wolfgang
(86) International application number: PCT/EP2003/014344
(87) International publication number: WO 2004/054601

(56) References cited:
- EP-A2- 0 796 913
- EP-A2- 0 796 913
- EP-A2- 1 279 744
- EP-A2- 1 279 744
- DATABASE WPI Section Ch, Week 200011 Derwent Publications Ltd., London, GB; Class B04, AN 2000-126652 XP002261387 & WO 99/67637 A1 (MERCK PATENT GMBH) 29 December 1999 (1999-12-29)
- DATABASE WPI Section Ch, Week 200217 Derwent Publications Ltd., London, GB; Class B04, AN 2002-130151 XP002261388 & WO 01/18210 A1 (GENENTECH INC) 15 March 2001 (2001-03-15)
- DATABASE WPI Section Ch, Week 200214 Derwent Publications Ltd., London, GB; Class B04, AN 2002-106464 XP002261389 & WO 01/96371 A2 (DEVELOGEN AG) 20 December 2001 (2001-12-20)
- DATABASE WPI Section Ch, Week 200017 Derwent Publications Ltd., London, GB; Class B04, AN 2000-195157 XP002261390 & WO 00/06087 A2 (TULARIK INC) 10 February 2000 (2000-02-10)
- DATABASE WPI Section Ch, Week 200163 Derwent Publications Ltd., London, GB; Class B04, AN 2001-565582 XP002261391 & WO 01/66706 A1 (HUMAN GENOME SCI INC) 13 September 2001 (2001-09-13)
- DATABASE WPI Section Ch, Week 200224 Derwent Publications Ltd., London, GB; Class B04, AN 2002-188506 XP002261392 & WO 02/05810 A1 (JOSLIN DIABETES CENT) 24 January 2002 (2002-01-24)
- DATABASE UNIPROT [Online] EMBL-EBI; Phosphatidylinositol 4-kinase beta isoform 1 June 2001 (2001-06-01), EMBL-EBI: "FWD - CG7004" XP002301946 retrieved from WWW.EBI.AC.UK accession no. Q9BKJ2 Database accession no. Q9BKJ2
- DATABASE UNIPROT [Online] EMBL-EBI; CG7004-PA (Cg7004-pb) (LP07057p). 1 May 2000 (2000-05-01), EMBL/EBI: "FWD - CG7004" XP002301947 retrieved from WWW.EBI.AC.UK accession no. Q9W0R8 Database accession no. Q9W0R8
- DATABASE UNIPROT [Online] EMBL-EBI; Phosphatidylinositol 4-kinase beta (EC 2.7.1.67) 29 March 2004 (2004-03-29), EMBL: "P4KB_HUMAN" XP002301948 retrieved from WWW.EBI.AC.UK accession no. Q9UBF8 Database accession no. Q9UBF8
- DATABASE REFSEQ [Online] NCBI; Phosphatidylinositol 4-kinase beta (EC 2.7.1.67) 23 August 2004 (2004-08-23), REFSEQ: XP002301949 retrieved from WWW.EBI.AC.UK accession no. NP_002642 Database accession no. NP_002642
- DATABASE REFSEQ [Online] NCBI; Phosphatidylinositol 4-kinase beta mRNA 23 August 2004 (2004-08-23), REFSEQ: XP002301950 retrieved from WWW.EBI.AC.UK accession no. NM_002651 Database accession no. NM_002651
- DATABASE GENBANK [Online] NCBI 31 October 2000 ANONYMOUS: 'Homo sapiens phosphatidylinositol 4-kinase, catalytic, beta' Retrieved from www.ncbi.nlm.nih.gov/entrez/ Database accession no. NM_002651
- DATABASE GENBANK REVISION HISTORY [Online] NCBI 17 November 2002 ANONYMOUS Retrieved from /www.ncbi.nlm.nih.gov/sviewer/ Database accession no. XM_196305
- DATABASE GENBANK REVISION HISTORY [Online] NCBI 15 November 2002 ANONYMOUS Retrieved from /www.ncbi.nlm.nih.gov/sviewer/ Database accession no. XM_205921
- DATABASE GENBANK [Online] NCBI 31 October 2000 ANONYMOUS: 'Homo sapiens phosphatidylinositol 4-kinase, catalytic, beta' Retrieved from www.ncbi.nlm.nih.gov/entrez/ Database accession no. NM_002651
- DATABASE GENBANK REVISION HISTORY [Online] NCBI 17 November 2002 ANONYMOUS: 'XM_196305' Retrieved from /www.ncbi.nlm.nih.gov/sviewer/ Database accession no. XM_196305
- DATABASE GENBANK REVISION HISTORY [Online] NCBI 15 November 2002 ANONYMOUS: 'XM_205921' Retrieved from /www.ncbi.nlm.nih.gov/sviewer/ Database accession no. XM_205921

## Description

This invention relates to the identification of (poly)peptides involved in the regulation of energy metabolism and/or metabolism of triglycerides using a *four wheel drive* (Gadfly Accession Number CG7004; referred to as *fwd*), homologous protein, and to the diagnosis of obesity and/or metabolic syndrome.

There are several metabolic diseases of human and animal metabolism, e.g., obesity and severe weight loss, that relate to energy imbalance where caloric intake versus energy expenditure is imbalanced. Obesity is one of the most prevalent metabolic disorders in the world. It is still a poorly understood human disease that becomes as a major health problem more and more relevant for western society. Obesity is defined as a body weight more than 20% in excess of the ideal body weight, frequently resulting in a significant impairment of health. It is associated with an increased mortality rate. Besides severe risks of illness, individuals suffering from obesity are often isolated socially.

Obesity is influenced by genetic, metabolic, biochemical, psychological, and behavioral factors, and can be caused by different reasons such as non-insulin dependent diabetes, increase in triglycerides, increase in carbohydrate bound energy and low energy expenditure. As such, it is a complex disorder that must be addressed on several fronts to achieve lasting positive clinical outcome.

Since obesity is not to be considered as a single disorder but as a heterogeneous group of conditions with (potential) multiple causes, it is also characterized by elevated fasting plasma insulin and an exaggerated insulin response to oral glucose intake (Koltermann O.G. et al., (1980) J. Clin. Invest 65: 1272-1284). A clear involvement of obesity in type 2 diabetes mellitus can be confirmed (Kopelman P.G., (2000) Nature 404: 635-643).

Triglycerides and glycogen are used as the body's fuel energy storage. Glycogen is a large branched polymer of glucose residues that is mainly stored in liver and muscle cells. Glycogen, synthesis and degradation is central to the control of the blood glucose level.

Triglycerides are stored in the cytoplasm of adipocytes. Adipocytes are specialized for the synthesis, storage and mobilization of triglycerides. The glycogen and triglyceride metabolism is highly regulated and their interplay is essential for the energy homeostasis of the body. A high glucose level in the adipose cell results in the synthesis of triglycerides as fuel store. A low intracellular glucose level leads to a release of fatty acids, which can be used as substrates for the beta-oxidation to generate energy. Glycogen levels in cells are more variable than triglyceride levels because the turnover of glycogen is higher. Triglycerides are used as long term energy donors once the glycogen stores run low.

Insulin amongst other hormones plays a key role in the regulation of the fuel metabolism. High blood glucose levels stimulate the secretion of insulin by pancreatic beta-cells. Insulin leads to the storage of glycogen and triglycerides and to the synthesis of proteins. The entry of glucose into muscles and adipose cells is stimulated by insulin.

In patients who suffer from diabetes mellitus either the amount of insulin produced by the pancreatic islet cells is to low (Diabetes Type 1 or insulin dependent diabetes mellitus IDDM) or liver and muscle cells loose their ability to respond to normal blood insulin levels (insulin resistance). In the next stage pancreatic cells become unable to produce sufficient amounts of insulin (Diabetes Type II or non insulin dependent diabetes mellitus NIDDM).

Hyperlipidemia and elevation of free fatty acids correlate clearly with the metabolic syndrome, which is defined as the linkage between several diseases, including obesity an insulin resistance. This often occurs in the same patients and are major risk factors for development of type 2 diabetes and cardiovascular disease. It was suggested that the control of lipid levels and glucose levels is required to treat type 2 diabetes, heart disease, and other occurances of metabolic syndrome (see, for example, Santomauro A.T. et al., (1999) Diabetes, 48: 1836-1841 and Lakka H.M. et al., (2002) JAMA 288: 2709-2716).

Pancreatic beta-cells secrete insulin in response to blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus type I or LADA (latent autoimmue diabetes in adults (Pozzilli & Di Mario, 2001, Diabetes Care. 8: 1460-1467) beta-cells are being destroyed due to autoimmune attack. The amount of insulin produced by the remaining pancreatic islet cells is too low, resulting in elevated blood glucose levels (hyperglycemia). In diabetes type 2 liver and muscle cells loose their ability to respond to normal blood insulin levels (insulin resistance). High blood glucose levels (and also high blood lipid levels) in turn lead to an impairment of beta-cell function and to an increase in beta-cell apoptosis.

Diabetes is a very disabling disease, because today's common anti-diabetic drugs do not control blood sugar levels well enough to completely prevent the occurrence of high and low blood sugar levels. Out of range blood sugar levels are toxic and cause long-term complications like for example renopathy, retinopathy, neuropathy and peripheral vascular disease. There is also a host of related conditions, such as obesity, hypertension, heart disease and hyperlipidemia , for which persons with diabetes are substantially at risk.

Apart from the impaired quality of life for the patients, the treatment of diabetes and its long term complications presents an enormous financial burden to our healthcare systems with rising tendency. Thus, for the treatment of, type I and type II diabetes as well as for latent autoimmune diabetes in adults (LADA) there is a strong need in the art to identify factors that induce regeneration of pancreatic insulin producing beta-cells. These factors could restore normal function of the endocrine pancreas once its function is impaired or event could prevent the development or progression of diabetes type I, diabetes type II, or LADA.

The concept of 'metabolic syndrome' (syndrome x, insulin-resistance syndrome, deadly quartet) was first described 1966 by Camus and reintroduced 1988 by Reaven (Camus J.P., (1966) Rev Rhum Mal Osteoartic 33: 10-14; Reaven G.M. et al., (1988) Diabetes, 37: 1595-1607). Today metabolic syndrome is commonly defined as clustering of cardiovascular risk factors like hypertension, abdominal obesity, high blood levels of triglycerides and fasting glucose as well as low blood levels of HDL cholesterol. Insulin resistance greatly increases the risk of developing the metabolic syndrome (Reaven G., (2002) Circulation 106: 286-288). The metabolic syndrome often precedes the development of type II diabetes and cardiovascular disease (Lakka H.M. et al., 2002, supra).

Hyperlipidemia and elevation of free fatty acids correlate clearly with the metabolic syndrome, which is defined as the linkage between several diseases, including obesity an insulin resistance. This often occurs in the same patients and is a major risk factor for development of Type 2 diabetes and cardiovascular disease. It was suggested that the control of lipid levels and glucose levels is required to treat Type 2 Diabetes, heart disease, and other occurances of metabolic syndrome (see, for example, Santomauro A.T. et al., (1999) Diabetes, 48:1836-1841).

The molecular factors regulating food intake and body weight balance are incompletely understood. Even if several candidate genes have been described which are supposed to influence the homeostatic system(s) that regulate body mass/weight, like leptin or the peroxisome proliferator-activated receptor-gamma co-activator, the distinct molecular mechanisms and/or molecules influencing obesity or body weight/body mass regulations are not known. In addition, several single-gene mutations resulting in obesity have been described in mice, implicating genetic factors in the etiology of obesity (Friedman J.M. and Leibel R.L., (1990) Cell 69: 217-220). In the ob mouse a single gene mutation (obese) results in profound obesity, which is accompanied by diabetes (Friedman J.M. et. al., (1991) Genomics 11: 1054-1062).

Therefore, the technical problem underling the present invention was to provide methods for identifying (poly)peptides involved in the regulation of energy homeostasis or/and metabolism of triglycerides in a mammal and for diagnosing metabolic syndrome or obesity. The solution to said technical problem is achieved by providing the embodiments characterized in the claims.

EP 0 796 913 discloses a human NPIK gene and the respective protein which can be used for treating hereditary diseases and cancer. However, there is no information about the involvement of the described NPIK in energy homeostasis and metabolism of triglycerides.

The fwd homologous polypeptide PIK4CB (NCBI Genbank Accession No. NM_002642) and the respective CDNA (NM_002651) were known in the art.

So far, it has not been described that PIK4CB is involved in the regulation of energy homeostasis and body-weight regulation and related disorders, and thus, no functions in metabolic diseases and dysfunctions and other diseases as listed above have been discussed.

Before the present methods are described, it is understood that this invention is not limited to the particular methodology, protocols, cell lines, vectors, and reagents described as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are for the purpose of describing and disclosing the cell lines, vectors, and methodologies that are reported in the publications which might be used in connection with the invention. Nothing herein is to be construed as an admission that the invention is not entitled to antedate such disclosure.

It is described herein that *fwd* homologous proteins (herein referred to as "proteins of the description" or "a protein of the description") are regulating the energy homeostasis and fat metabolism, especially the metabolism and storage of triglycerides, and polynucleotides, which identify and encode the proteins disclosed herein. Further described are vectors, host cells, and recombinant methods for producing the polypeptides and polynucleotides. Also described is the use of these compounds and effectors/modulators thereof, e.g. antibodies, in the diagnosis and study of metabolic diseases or dysfunctions, including obesity, and/or metabolic syndrome.

The synthesis of phosphatidylinositol 4,5-bisphosphate [Ptdlns(4,5)P₂], the immediate precursor of intracellular signals generated by calcium-mobilizing hormones and growth factors, is initiated by the conversion of phosphatidylinositol to phosphatidylinositol 4-phosphate [Ptdlns(4)P] by phosphatidylinositol 4-kinase (Ptdlns 4-kinase) (Meyers R. and Cantley L.C., (1997) J. Biol. Chem. 272: 4384-4390). Targeting of Golgi-specific pleckstrin homology domains of oxysterol binding protein (OSBP) involves both Ptdlns 4-kinase-dependent and -independent components (Levine T.P. and Munro S., (2002) Curr Biol 12: 695-704).

Biochemical analyses indicated that phosphatidyl inositol 4-kinase beta (Pl4Kbeta, PIK4CB) is a type III enzyme that is sensitive to wortmannin (Meyers R. and Cantley L.C., supra). Pl4Kbeta is localized in the cytosol and also present in the Golgi region (Wong K. et al., (1997) J Biol Chem 272: 13236-13241). PI4Kbeta is ubiquitously expressed, with highest expression in heart, pancreas, and skeletal muscle.

PI4Kbeta (PIK4CB, PI4K type III) was primarily found in the Golgi, but it was also present in the walls of numerous large perinuclear vesicles. Co-expression of a catalytically inactive PI4Kbeta inhibited the development of this vesicular phenotype. PI4Kbeta is involved in vesicular trafficking (Zhao X. et al., (2001) J Biol Chem 276: 40183-40189).

*Fwd* homologous proteins and nucleic acid molecules coding therefore are obtainable from insect or vertebrate species, e.g. mammals or birds. Also disclosed are nucleic acids encoding the human *fwd* homologs as described in Table 1.

A nucleic acid molecule encoding a polypeptide contributing to regulating the energy homeostasis and the metabolism of triglycerides and glycogen, comprises
(a) the nucleotide sequence of Drosophila *fwd, Pp2C1, Adk3,* CG3860, *Cdk4,* CG7134, or *Eip75B,* human *fwd*, *Pp2C1*, *Adk3*, CG3860, *Cdk4,* CG7134, or *Eip75B* homologous nucleic acids, particularly the nucleic acids as described in Table 1, and/or a sequence complimentary thereto,
(b) a nucleotide sequence which hybridizes at 50°C in a solution containing 1 x SSC and 0.1% SDS to a sequence of (a),
(c) a sequence corresponding to the sequences of (a) or (b) within the degeneration of the genetic code,
(d) a sequence which encodes a polypeptide which is at least 85%, preferably at least 90%, more preferably at least 95%, more preferably at least 98% and up to 99,6% identical to the amino acid sequences of the *fwd, Pp2C1, Adk3,* CG3860, *Cdk4,* CG7134, or *Eip75B* homologous protein, preferably of the human *fwd, Pp2C1, Adk3,* CG3860, *Cdk4,* CGG7134, or *Eip75B* homologous protein, particularly a protein as described in Table 1,
(e) a sequence which differs from the nucleic acid molecule of (a) to (d) by mutation and wherein said mutation causes an alteration, deletion, duplication and/or premature stop in the encoded polypeptide or
(f) a partial sequence of any of the nucleotide sequences of (a) to (e) having a length of 15-25 bases, preferably 25-35 bases, more preferably 35-50 bases and most preferably at least 50 bases.

The invention is based on the finding that the *fwd* homologous polypeptide PIK4CB and the polynucleotide encoding therefore, are involved in the regulation of triglyceride storage and therefore energy homeostasis. Any information about other *fwd* homologous proteins, Pp2C1, Adk3, CG3860, Cdk4, CG7134 or Eip75B homologous proteins is provided for comparison only. Also described is the use of compositions comprising *fwd*, *Pp2C1*, *Adk3*, CG3860, *Cdk4*, CG7134, or *Eip75B* homologous polypeptides and polynucleotides as well as modulators/effectors thereof for the diagnosis and study of metabolic diseases or dysfunctions, including obesity and/or metabolic syndrome.

Accordingly, described are genes with novel functions in body-weight regulation, energy homeostasis, metabolism, and obesity, fragments of said genes, polypeptides encoded by said genes or fragments thereof, and effectors e.g. antibodies.

The ability to manipulate and screen the genomes of model organisms such as the fly Drosophila melanogaster provides a powerful tool to analyze biological and biochemical processes that have direct relevance to more complex vertebrate organisms due to significant evolutionary conservation of genes, cellular processes, and pathways (see, for example, Adams M.D. et al., (2000) Science 287: 2185-2195). Identification of novel gene functions in model organisms can directly contribute to the elucidation of correlative pathways in mammals (humans) and of methods of modulating them. A correlation between a pathology model (such as changes in triglyceride levels as indication for metabolic syndrome including obesity) and the modified expression of a fly gene can identify the association of the human ortholog with the particular human disease.

A forward genetic screen is performed in fly displaying a mutant phenotype due to misexpression of a known gene (see, St. Johnston D., (2002) Nat Rev Genet 3: 176-188 ; Rorth P., (1996) Proc Natl Acad Sci USA 93: 12418-12422). A genetic screen was used to identify mutations of *fwd*, *Pp2C1*, *Adk3,* CG3860, *Cdk4,* CG7134, or *Eip75B* genes that cause changes in the body weight which are reflected by a significant change of triglyceride levels. Additionally glycogen levels are analysed.

Obese people mainly show a significant increase in the content of triglycerides. Triglycerides are the most efficient storage for energy in cells. In order to isolate genes with a function in energy homeostasis, several thousand proprietary and publicly available EP-lines were tested for their triglyceride content after a prolonged feeding period (see Examples and Figures for more detail). Lines with significantly changed triglyceride content were selected as positive candidates for further analysis. The change of triglyceride content due to the loss of a gene function suggests gene activities in energy homeostasis in a dose dependent manner that controls the amount of energy stored as triglycerides.

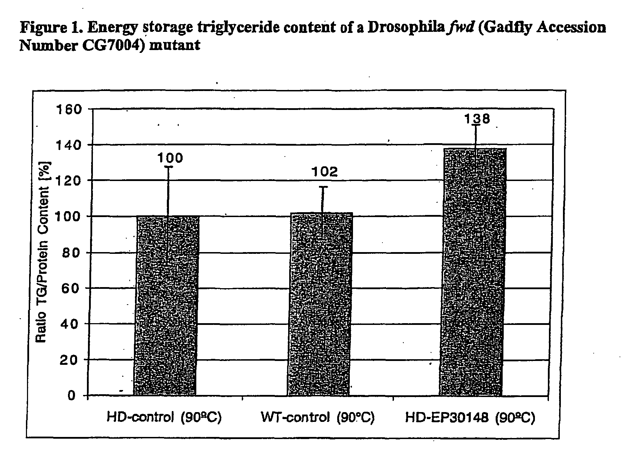

The content of triglycerides of a pool of flies with the same genotype was analyzed after prolonged feeding using a triglyceride assay. Male flies homozygous, hemizygous, or heterozygous for the integration of vectors for Drosophila EP-lines were analyzed in an assay measuring the triglyceride contents of these flies, illustrated in more detail in the Examples section. The results of the triglyceride content analysis are shown in Figures 1, 5, 8, 12, 16, 18, and 20, respectively.

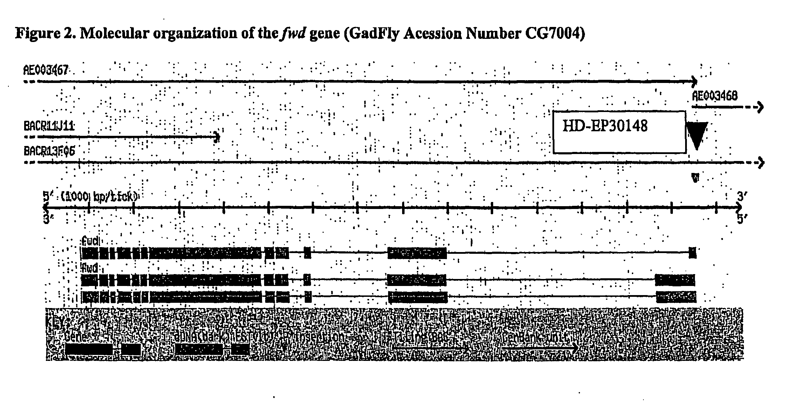

Genomic DNA sequences were isolated that are localized adjacent to the EP vector integration. Using those isolated genomic sequences public databases like Berkeley Drosophila Genome Project (GadFly; see also FlyBase (1999) Nucleic Acids Research 27: 85-88) were screened thereby identifying the integration sites of the vectors, and the corresponding genes, described in more detail in the Examples section. The molecular organization of the genes is shown in Figures 2, 6, 9, 13, 17, 19, and 21, respectively.

The function of *Eip75B* in metabolic disorders is further validated by data obtained from an additional screen. For example, an additional screen using Drosophila mutants with modifications of the eye phenotype identified a modification of UCP activity by *Eip75B,* thereby leading to an altered mitochondrial activity. These findings suggest the presence of similar activities of these described homologous proteins in humans that provides insight into diagnosis, treatment, and prognosis of metabolic disorders.

The Drosophila genes and proteins encoded thereby with functions in the regulation of triglyceride metabolism were further analysed in publicly available sequence databases (see Examples for more detail) and mammalian homologs were identified.

The function of the mammalian homologs in energy homeostasis was further validated by analyzing the expression of the transcripts in different tissues and by analyzing the role in adipocyte differentiation. Expression profiling studies (see Examples for more detail) confirm the particular relevance of the described proteins as regulators of energy metabolism in mammals. Further, we show that the described proteins are regulated by fasting and by genetically induced obesity. We used mouse models of insulin resistance and/or diabetes, such as mice carrying gene knockouts in the leptin pathway (for example, ob (lepfin) or db (leptin receptor) mice) to study the expression of the described proteins. Such mice develop typical symptoms of diabetes, show hepatic lipid accumulation and frequently have increased plasma lipid levels (see Bruning J.C. et al., (1998) Mol. Cell. 2: 559-569).

Microarrays are analytical tools routinely used in bioanalysis. A microarray has molecules distributed over, and stably associated with, the surface of a solid support. The term "microarray" refers to an arrangement of a plurality of polynucleotides, polypeptides, antibodies, or other chemical compounds on a substrate. Microarrays of polypeptides, polynucleotides, and/or antibodies have been developed and find use in a variety of applications, such as monitoring gene expression, drug discovery, gene sequencing, gene mapping, bacterial identification, and combinatorial chemistry. One area in particular in which microarrays find use is in gene expression analysis (see Example 6). Array technology can be used to explore the expression of a single polymorphic gene or the expression profile of a large number of related or unrelated genes. When the expression of a single gene is examined, arrays are employed to detect the expression of a specific gene or its variants. When an expression profile is examined, arrays provide a platform for identifying genes that are tissue specific, are affected by a substance being tested in a toxicology assay, are part of a signaling cascade, carry out housekeeping functions, or are specifically related to a particular genetic predisposition, condition, disease, or disorder.

Microarrays may be prepared, used, and analyzed using methods known in the art (see for example, Brennan T.M., (1995) U.S. Patent No. US5474796; Schena M. et al., (1996) Proc. Natl. Acad. Sci. USA 93: 10614-10619; Baldeschwieler et al., (1995) PCT application WO9525116; Shalon T.D. and Brown P.O., (1995) PCT application WO9535505; Heller R.A. et al., (1997) Proc. Natl. Acad. Sci. USA 94: 2150-2155; Heller M.J. and Tu E., (1997) U.S. Patent No. US5605662). Various types of microarrays are well known and thoroughly described in Schena M., ed. (1999); DNA Microarrays: A Practical Approach, Oxford University Press, London.

Oligonucleotides or longer fragments derived from any of the polynucleotides described herein may be used as elements on a microarray. The microarray can be used in transcript imaging techniques, which monitor the relative expression levels of large numbers of genes simultaneously as described below. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents, which are highly effective and display the fewest side effects may be selected for a patient based on his/her pharmacogenomic profile.

As determined by microarray analysis, phosphatidylinositol 4-kinase, catalytic, beta polypeptide (PIK4CB), adenylate kinase 3 like 1 (AK3L1), adenylate kinase 3 (AK3), oxysterol binding protein-like 1A (OSBPL1A), and oxysterol binding protein-like 2 (OSBPL2) are strong candidates for the manufacture of a pharmaceutical composition and a medicament for the treatment of conditions related to human metabolism, such as obesity, diabetes, and/or metabolic syndrome.

In another embodiment, we found that triglyceride levels were significantly increase in Nr1d-1 overexpressing cells during adipocyte differentiation. Glycogen levels in cells were more variable than triglyceride levels because the turnover of glycogen is higher. Glucose is taken up by the cells rapidly and stored in the form of glycogen. This energy storage is then used as a first quick response to the metabolic demands of the cell. During differentiation of adipocytes, glucose uptake in Nr1d-1 overexpressing cells is significantly increased. In another embodiment, triglyceride levels were significantly decreased in cells without functional Nr1d1 during adipocyte differentiation. Insulin stimulated lipid synthesis levels were significantly decreased during adipocyte differentiation in cells without functional Nr1d1. These findings show that the Nr1d1 plays a key role in central metabolic pathways in the cell.

Also described are polynucleotides that encode the described proteins and homologous proteins. Accordingly, any nucleic acid sequence, which encodes the amino acid sequences of the described proteins and homologous proteins, can be used to generate recombinant molecules that express the described proteins and homologous proteins. A nucleic acid encoding Drosophila *fwd*, *Pp2C1*, *Adk3*, CG3860, *Cdk4,* CG7134, or *Eip75B* or human *fwd, Pp2C1, Adk3,* CG3860, *Cdk4*, CG7134, or *Eip75B* homologous proteins, preferably a human homologous protein is described in

Table 1. It will be appreciated by those skilled in the art that as a result of the degeneracy of the genetic code, a multitude of nucleotide sequences encoding the proteins, some bearing minimal homology to the nucleotide sequences of any known and naturally occurring gene, may be produced.

Also described are polynucleotide sequences that are capable of hybridizing to the claimed nucleotide sequences, and in particular, those of the polynucleotide encoding the described proteins, under various conditions of stringency. Hybridization conditions are based on the melting temperature (Tm) of the nucleic acid binding complex or probe, as described in Wahl G.M. et al. (1987: Methods Enzymol. 152: 399-407) and Kimmel A.R. (1987; Methods Enzymol. 152: 507-511), and may be used at a defined stringency. Preferably, hybridization under stringent conditions means that after washing for 1 h with 1 x SSC and 0.1 % SDS at 50°C, preferably at 55°C, more preferably at 62°C and most preferably at 65°C, particularly for 1 h in 0.2 x SSC and 0.1 % SDS at 50°C, preferably at 55°C, more preferably at 62°C and most preferably at 65°C, a positive hybridization signal is observed. Altered nucleic acid sequences encoding the described proteins include deletions, insertions or substitutions of different nucleotides resulting in a polynucleotide that encodes the same or a functionally equivalent protein.

The encoded proteins may also contain deletions, insertions or substitutions of amino acid residues, which produce a silent change and result in functionally equivalent proteins. Deliberate amino acid substitutions may be made on the basis of similarity in polarity, charge, solubility, hydrophobicity, hydrophilicity, and/or the amphipathic nature of the residues as long as the biological activity of the protein is retained.

Also described are alleles of the genes encoding the described proteins. As used herein, an 'allele' or 'allelic sequence' is an alternative form of the gene, which may result from at least one mutation in the nucleic acid sequence. Alleles may result in altered mRNAs or polypeptides whose structures or function may or may not be altered. Any given gene may have none, one or many allelic forms. Common mutational changes, which give rise to alleles, are generally ascribed to natural deletions, additions or substitutions of nucleotides. Each of these types of changes may occur alone or in combination with the others, one or more times in a given sequence.

The nucleic acid sequences encoding the described proteins and homologous proteins may be extended utilizing a partial nucleotide sequence and employing various methods known in the art to detect upstream sequences such as promoters and regulatory elements. For example, one method which may be employed, 'restriction-site' PCR, uses universal primers to retrieve unknown sequence adjacent to a known locus (Sarkar G. et al., (1993) PCR Methods Applic. 2: 318-322). Inverse PCR may also be used to amplify or extend sequences using divergent primers based on a known region (Triglia T. et al., (1988) Nucleic Acids Res. 16: 8186). Another method which may be used is capture PCR which involves PCR amplification of DNA fragments adjacent to a known sequence in human and yeast artificial chromosome DNA (Lagerstrom M. et al., (1991) PCR Methods Applic. 1: 111-119). Another method which may be used to retrieve unknown sequences is that of Parker J.D. et al., (1991) Nucleic Acids Res. 19: 3055-3060. Additionally, one may use PCR, nested primers, and PROMOTERFINDER libraries to walk in genomic DNA (Clontech, Palo Alto, Calif.). This process avoids the need to screen libraries and is useful in finding intron/exon junctions.

In order to express a biologically active protein, the nucleotide sequences encoding the proteins or functional equivalents, may be inserted into appropriate expression vectors, i.e., a vector which contains the necessary elements for the transcription and translation of the inserted coding sequence. Methods, which are well known to those skilled in the art, may be used to construct expression vectors containing sequences encoding the proteins and the appropriate transcriptional and translational control elements. Regulatory elements include for example a promoter, an initiation codon, a stop codon, a mRNA stability regulatory element, and a polyadenylation signal. Expression of a polynucleotide can be assured by (i) constitutive promoters such as the cytomegalovirus (CMV) promoter/enhancer region, (ii) tissue specific promoters such as the insulin promoter (see, Soria B. et al., (2000) Diabetes 49: 157-162), SOX2 gene promotor (see Li M. et al., (1998) Curr. Biol. 8: 971-974), Msi-1 promotor (see Sakakibara S. and Okano H., (1997) J. Neuroscience 17: 8300-8312), alpha-cardia myosin heavy chain promotor or human atrial natriuretic factor promotor (Klug M.G. et al., (1996) J. Clin. Invest 98: 216-224; Wu J. et al., (1989) J. Biol. Chem. 264: 6472-6479) or (iii) inducible promoters such as the tetracycline inducible system. Expression vectors can also contain a selection agent or marker gene that confers antibiotic resistance such as the neomycin, hygromycin or puromycin resistance genes. These methods include in vitro recombinant DNA techniques, synthetic techniques, and in vivo genetic recombination. Such techniques are described in Sambrook, J. et al., (1989) Molecular Cloning, A Laboratory Manual, Cold Spring Harbor Press, Plainview, N.Y. and Ausubel, F.M. et al. (1989) Current Protocols in Molecular Biology, John Wiley & Sons, New York, N.Y.

Further, natural, modified or recombinant nucleic acid sequences encoding the described proteins and homologous proteins may be ligated to a heterologous sequence to encode a fusion protein.

A variety of expression vector/host systems may be utilized to contain and express sequences encoding the proteins or fusion proteins. These include, but are not limited to, micro-organisms such as bacteria transformed with recombinant bacteriophage, plasmid or cosmid DNA expression vectors; yeast transformed with yeast expression vectors; insect cell systems infected with virus expression vectors (e.g., baculovirus, adenovirus, adeno-associated virus, lentiverus, retrovirus); plant cell systems transformed with virus expression vectors (e.g., cauliflower mosaic virus, CaMV; tobacco mosaic virus, TMV) or with bacterial expression vectors (e.g., Ti or PBR322 plasmids); or animal cell systems.

The presence of polynucleotide sequences in a sample can be detected by DNA-DNA or DNA-RNA hybridization and/or amplification using probes or portions or fragments of said polynucleotides. Nucleic acid amplification based assays involve the use of oligonucleotides or oligomers based on the sequences specific for the gene to detect transformants containing DNA or RNA encoding the corresponding protein. As used herein 'oligonudeotides' or 'oligomers' refer to a nucleic acid sequence of at least about 10 nucleotides and as many as about 60 nucleotides, preferably about 15 to 30 nucleotides, and more preferably about 20-25 nucleotides, which can be used as a probe or amplimer.

A wide variety of labels and conjugation techniques are known by those skilled in the art and may be used in various nucleic acid and amino acid assays. Means for producing labeled hybridization or PCR probes for detecting polynucleotide sequences include oligo-labeling, nick translation, end-labeling of RNA probes, PCR amplification using a nucleotide, or enzymatic synthesis. These procedures may be conducted using a variety of commercially available kits (Pharmacia & Upjohn, (Kalamazoo, Mich.); Promega (Madison Wis.); and U.S. Biochemical Corp., (Cleveland, Ohio).

The presence of the described proteins in a sample can be determined by immunological methods or activity measurement. A variety of protocols for detecting and measuring the expression of proteins, using either polyclonal or monoclonal antibodies specific for the Protein or reagents for determining protein activity are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on the protein is preferred, but a competitive binding assay may be employed. These and other assays are described, among other places, in Hampton, R. et al. (1990; Serological Methods, a Laboratory Manual, APS Press, St Paul, Minn.) and Maddox, D.E. et al. (1983; J. Exp. Med. 158: 1211-1226).

Suitable reporter molecules or labels, which may be used, include radionuclides, enzymes, fluorescent, chemiluminescent or chromogenic agents as well as substrates, co-factors, inhibitors, magnetic particles, and the like.

Host cells transformed with nucleotide sequences encoding a protein may be cultured under conditions suitable for the expression and recovery of said protein from cell culture. The protein produced by a recombinant cell may be secreted or contained intracellularly depending on the sequence or/and the vector used. As will be understood by those of skill in the art, expression vectors containing polynucleotides, which encode the protein may be designed to contain signal sequences, which direct secretion of the protein through a prokaryotic or eukaryotic cell membrane. Other recombinant constructions may be used to join sequences encoding the protein to nucleotide sequence encoding a polypeptide domain, which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAG extension/affinity purification system (Immunex Corp., Seattle, Wash.) The inclusion of cleavable linker sequences such as those specific for Factor XA or Enterokinase (Invitrogen, San Diego, Calif.) between the purification domain and the desired protein may be used to facilitate purification.

### Diagnostics

The data disclosed in this document show that the described nucleic acids and proteins and modulator/effector molecules thereof are useful in diagnostic applications implicated in metabolic syndrome including obesity. Hence, diagnostic uses for the proteins and nucleic acids and modulators/effectors thereof are, for example but not limited to, the following: (i) small molecule drug target, (ii) antibody target (diagnostic), (iii) diagnostic and/or prognostic marker, and (v) research tools.

The nucleic acids and proteins and modulators/effectors thereof are useful in diagnostic applications implicated in various applications as described below.

The described nucleic acids or fragments thereof, may further be useful in diagnostic applications, wherein the presence or amount of the nucleic acids or the proteins are to be assessed. Further antibodies that bind immunospecifically to the described substances may be used in diagnostic methods.

Antibodies may include, but are not limited to, polyclonal, monoclonal, chimeric single chain, Fab fragments, and fragments produced by a Fab expression library.

For the production of antibodies, various hosts including goats, rabbits, rats, mice, humans, and others, may be immunized by injection with the protein or any fragment or oligopeptide thereof which has immunogenic properties. Depending, on the host species, various adjuvants may be used to increase immunological response. It is preferred that the peptides, fragments or oligopeptides used to induce antibodies to the protein have an amino acid sequence consisting of at least five amino acids, and more preferably at least 10 amino acids.

Monoclonal antibodies to the proteins may be prepared using any technique that provides for the production of antibody molecules by continuous cell lines in culture. These include, but are not limited to, the hybridoma technique, the human B-cell hybridoma technique, and the EBV-hybridoma technique (Köhler, G. and Milstein C., (1975) Nature 256: 495-497; Kozbor, D. et al., (1985) J. Immunol. Methods 81: 31-42; Cote, R.J. et al., (1983) Proc. Natl. Acad. Sci. 80: 2026-2030; Cole, S.P. et al., (1984) Mol. Cell Biochem. 62: 109-120).

In addition, techniques developed for the production of 'chimeric antibodies', the splicing of mouse antibody genes to human antibody genes to obtain a molecule with appropriate antigen specificity and biological activity can be used (Morrison, S.L. et al., (1984) Proc. Natl. Acad. Sci. USA 81: 6851-6855; Neuberger, M.S. et al., (1984) Nature 312: 604-608; Takeda, S. et al., (1985) Nature 314: 452-454). Alternatively, techniques described for the production of single chain antibodies may be adapted, using methods known in the art, to produce single chain antibodies specific for the described proteins and homologous proteins. Antibodies with related specificity, but of distinct idiotypic composition, may be generated by chain shuffling from random combinatorial immunoglobulin libraries (Kang, A.S. et al., (1991) Proc. Natl. Acad. Sci. USA 88: 11120-11123). Antibodies may also be produced by inducing in vivo production in the lymphocyte population or by screening recombinant immunoglobulin libraries or panels of highly specific binding reagents as disclosed in the literature (Orlandi, R. et al. (1989) Proc. Natl. Acad. Sci. USA 86: 3833-3837; Winter, G. and Milstein C., (1991) Nature 349: 293-299).

Antibody fragments which contain specific binding sites for the proteins may also be generated. For example, such fragments include, but are not limited to, the F(ab')₂ fragments which can be produced by Pepsin digestion of the antibody molecule and the Fab fragments which can be generated by reducing the disulfide bridges of F(ab')₂ fragments. Alternatively, Fab expression libraries may be constructed to allow rapid and easy identification of monoclonal Fab fragments with the desired specificity (Huse W.D. et al. (1989) Science 254: 1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between the protein and its specific antibody. A two-site, monoclonal-based immunoassay utilizing monoclonal antibodies reactive to two non-interfering protein epitopes are preferred, but a competitive binding assay may also be employed (Maddox, supra).

Nucleic acid modulator/effector molecules, may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences. Such DNA sequences may be incorporated into a variety of vectors with suitable RNA polymerase promoters such as T7 or SP6. Alternatively, these cDNA constructs that synthesize antisense RNA constitutively or inducibly can be introduced into cell lines, cells or tissues. RNA molecules may be modified to increase intracellular stability and half-life. Possible modifications include, but are not limited to, the addition of flanking sequences at the 5' and/or 3' ends of the molecule or modifications in the nucleobase, sugar and/or phosphate moieties, e.g. the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Antibodies which specifically bind to the proteins may be used for the diagnosis of conditions or diseases characterized by or associated with over- or under-expression of the described proteins and homologous proteins. Diagnostic assays include methods which utilize the antibody and a label to detect the protein in human body fluids or extracts of cells or tissues. The antibodies may be used with or without modification, and may be labeled by joining them, either covalently or non-covalently, with a reporter molecule. A wide variety of reporter molecules which are known in the art may be used several of which are described above.

A variety of protocols including ELISA, RIA, and FACS for measuring proteins are known in the art and provide a basis for diagnosing altered or abnormal levels of gene expression. Normal or standard values for gene expression are established by combining body fluids or cell extracts taken from normal mammalian subjects, preferably human, with antibodies to the protein under conditions suitable for complex formation. The amount of standard complex formation may be quantified by various methods, but preferably by photometric means. Quantities of protein expressed in control and disease, samples e.g. from biopsied tissues are compared with the standard values. Deviation between standard and subject values establishes the parameters for diagnosing disease.

The polynucleotides specific for the described proteins and homologous proteins may be used for diagnostic purposes. The polynucleotides, which may be used, include oligonucleotide sequences, antisense RNA and DNA molecules, and PNAs. The polynucleotides may be used to detect and quantitate gene expression in biopsied tissues in which gene expression may be correlated with disease. The diagnostic assay may be used to distinguish between absence, presence, and excess gene expression, and to monitor regulation of protein levels during therapeutic intervention.

In one aspect, hybridization with probes which are capable of detecting polynucleotide sequences, including genomic sequences, encoding the proteins and homologous proteins or closely related molecules, may be used to identify nucleic acid sequences which encode the respective protein. The hybridization probes may be DNA or RNA and are preferably derived from the nucleotide sequence of the polynucleotides encoding the proteins or from a genomic sequence including promoter, enhancer elements, and introns of the naturally occurring gene. Hybridization probes may be labeled by a variety of reporter groups, for example, radionuclides such as ³²P or ³⁵S or enzymatic labels, such as alkaline phosphatase coupled to the probe via avidin/biotin coupling systems, and the like.

Polynucleotide sequences specific for the proteins and homologous nucleic acids may be used for the diagnosis of conditions or diseases, which are associated with the expression of the proteins. Such conditions or diseases are metabolic diseases and disorders, including obesity. Polynucleotide sequences specific for the described proteins and homologous proteins may also be used to monitor the progress of patients receiving treatment for metabolic diseases and disorders, including obesity. The polynucleotide sequences may be used qualitative or quantitative assays, e.g. in Southern or Northern analysis, dot blot or other membrane-based technologies; in PCR technologies; or in dip stick, pin, ELISA or chip assays utilizing fluids or tissues from patient biopsies to detect altered gene expression.

In a particular aspect, the nucleotide sequences specific for the proteins and homologous nucleic acids may be useful in assays that detect activation or induction of various metabolic diseases or dysfunctions, including metabolic syndrome and obesity. The nucleotide sequences may be labeled by standard methods, and added to a fluid or tissue sample from a patient under conditions suitable for the formation of hybridization complexes. After a suitable incubation period, the sample is washed and the signal is quantitated and compared with a standard value. The presence of altered levels of nucleotide sequences encoding the proteins and homologous proteins in the sample indicates the presence of the associated disease. Such assays may also be used to evaluate the efficacy of a particular therapeutic treatment regimen in animal studies, in clinical trials or in monitoring the treatment of an individual patient.

In order to provide a basis for the diagnosis of a disease associated with expression of the described proteins and homologous proteins, a normal or standard profile for expression is established. This may be accomplished by combining body fluids or cell extracts taken from normal subjects, either animal or human, with a sequence or a fragment thereof, which is specific for the nucleic acids encoding the proteins and homologous nucleic acids; under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for disease. Deviation between standard and subject values is used to establish the presence of disease. Once disease is established and a treatment protocol is initiated, hybridization assays may be repeated on a regular basis to evaluate whether the level of expression in the patient begins to approximate that, which is observed in the normal patient. The results obtained from successive assays may be used to show the efficacy of treatment over a period ranging from several days to months.

With respect to metabolic diseases such as described above the presence of an unusual amount of transcript in biopsied tissue from an individual may indicate a predisposition for the development of the disease or may provide a means for detecting the disease prior to the appearance of actual clinical symptoms. A more definitive diagnosis of this type may allow health professionals to employ preventative measures or aggressive treatment earlier thereby preventing the development or further progression of the metabolic diseases and disorders.

Additional diagnostic uses for oligonucleotides designed from the sequences encoding the proteins and homologous proteins may involve the use of PCR. Such oligomers may be chemically synthesized, generated enzymatically or produced from a recombinant source. Oligomers will preferably consist of two nucleotide sequences, one with sense orientation (5'.fwdarw.3') and another with antisense (3'.rarw.5'), employed under optimized conditions for identification of a specific gene or condition. The same two oligomers, nested sets of oligomers or even a degenerate pool of oligomers may be employed under less stringent conditions for detection and/or quantification of closely related DNA or RNA sequences.

The nucleic acid sequences may also be used to generate hybridization probes, which are useful for mapping the naturally occurring genomic sequence. The sequences may be mapped to a particular chromosome or to a specific region of the chromosome using well known techniques. Such techniques include FISH, FACS or artificial chromosome constructions, such as yeast artificial chromosomes, bacterial artificial chromosomes, bacterial P1 constructions or single chromosome cDNA libraries as reviewed in Price C.M., (1993) Blood Rev. 7: 127-134, and Trask B.J., (1991) Trends Genet. 7: 149-154. FISH (as described in Verma R.S. and Babu A., (1989) Human Chromosomes: A Manual of Basic Techniques, Pergamon Press, New York, N.Y.). The results may be correlated with other physical chromosome mapping techniques and genetic map data. Examples of genetic map data can be found in the 1994 Genome Issue of Science (265: 1981f). Correlation between the location of the gene encoding the proteins on a physical chromosomal map and a specfic disease or predisposition to a specific disease, may help to delimit the region of DNA associated with that genetic disease.

The nucleotide sequences may be used to detect differences in gene sequences between normal, carrier or affected individuals. An analysis of polymorphisms, e.g. single nucleotide polymorphisms may be carried out. Further, in situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known: New sequences can be assigned to chromosomal arms or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti R.A. et al., (1988) Nature 336: 577-580), any sequences mapping to that area may represent associated or regulatory genes for further investigation. The nucleotide sequences may also be used to detect differences in the chromosomal location due to translocation, inversion, etc. among normal, carrier or affected individuals.

The described proteins, their catalytic or immunogenic fragments or oligopeptides thereof, an in vitro model, a genetically altered cell or animal, can be used for screening libraries of compounds, e.g. peptides or low molecular weight organic compounds, in any of a variety of drug screening techniques. One can identify modulators/effectors, e.g. receptors, enzymes, proteins, ligands, or substrates that bind to, modulate or mimic the action of one or more of the proteins. The protein or fragment thereof employed in such screening may be free in solution, affixed to a solid support, borne on a cell surface, or located intracellulary. The formation of binding complexes, between the protein and the agent tested, may be measured. Agents could also, either directly or indirectly, influence the activity of the proteins.

For example the phosphatase activity of the proteins could be measured in vitro by using recombinantly expressed and purified CG7134 or *Pp2C1* homologous phosphatase or fragments thereof by making use of artificial substrates well known in the art, i.e. but not exclusively DiFMUP (Molecular Probes, Eugene, Oregon), which are converted to fluorophores or chromophores upon dephosphorylation. Alternatively, the dephosphorylation of physiological substrates of the phosphatases could be measured by making use of any of the well known screening technologies suitable for the detection of the phosphorylation status of their physiological substrates. For example, but not exclusively, the phosphorylation status of peptides derived from their physiological substrates can be monitored by binding of phosphoside specific antibodies resulting in an increase of the polarization of the complex.

In vivo, the enzymatic kinase activity of the unmodified polypeptides of *Cdk4, fwd*, or *Adk3* homologous, kinase towards a substrate can be enhanced by appropriate stimuli, triggering the phosphorylation of *Cdk4, fwd,* or *Adk3* homologous kinase. This may be induced in the natural context by extracellular or intracellular stimuli, such as signaling molecules or environmental influences. One may generate a system containing activated *Cdk4, fwd,* or *Adk3* homologous kinase, may it be an organisms, a tissue, a culture of cells or cell-free environment, by exogenously applying this stimulus or by mimicking this stimulus by a variety of the techniques, some of them described further below. A system containing activated *Cdk4*, *fwd*, or *Adk3* homologous kinase may be produced (i) for the purpose of diagnosis, study, prevention, and treatment of diseases and disorders related to body-weight regulation and thermogenesis, for example, but not limited to, metabolic diseases or dysfunctions, including metabolic syndrome, obesity, or diabetes, as well as related disorders such as eating disorder, cachexia, hypertension, coronary heart disease, hypercholesterolemia, dyslipidemia, osteoarthritis, gallstones, or liver fibrosis.

Methods for determining protein-protein interaction are well known in the art. For example binding of a fluorescently labeled peptide derived from the interacting protein to the described protein, or vice versa, could be detected by a change in polarisation. In case that both binding partners, which can be either the full length proteins as well as one binding partner as the full length protein and the other just represented as a peptide are fluorescently labeled, binding could be detected by fluorescence energy transfer (FRET) from one fluorophore to the other. In addition, a variety of commercially available assay principles suitable for detection of protein-protein interaction are well known in the art, for example but not exclusively AlphaScreen (PerkinElmer) or Scintillation Proximity Assays (SPA) by Amersham. Alternatively, the interaction of the described protein with cellular proteins could be the basis for a cell-based screening assay, in which both proteins are fluorescently labeled and interaction of both proteins is detected by analysing cotranslocation of both proteins with a cellular imaging reader, as has been developed for example, but not exclusively, by Cellomics or EvotecOAl. In all cases the two or more binding partners can be different proteins with one being the described protein, or in case of dimerization and/or oligomerization the described protein itself. Proteins, for which one target mechanism of interest, but not the only one, would be such protein-protein interaction are *fwd*, *Pp2C1*, *Adk3*, CG3860, *Cdk4*, CG7134, or *Eip75B* homologous proteins.

Assays for determining enzymatic activity of the proteins are well known in the art. Well known in the art are also a variety of assay formats to measure receptor-ligand binding or receptor downstream signalling.

Genetic reporter systems are widely used to study eukaryotic gene expression and cellular physiology. Applications include the study of receptor activity, transcription factors, intracellular signalling, mRNA processing, and protein folding. For example, the firefly luciferase is used as a reporter because the luciferase assay is very sensitive and rapid. Luciferase reporter assays are commercially available, e.g. from BD Bioscience, Promega, and Boehringer Mannheim. Other reporter genes can be also used to detect eukaryotic gene expression, like chloramphenicol acetyltransferase (CAT), beta-galactosidase (beta-Gal), or human placental alkaline phosphatase (SEAP).

Of particular interest are screening assays for agents that have a low toxicity for mammalian cells. The term "agent" as used herein describes any molecule, e.g. protein or pharmaceutical, with the capability of altering or mimicking the physiological function of one or more of the described proteins. Candidate agents encompass numerous chemical classes, though typically they are organic molecules, preferably small organic compounds having a molecular weight of more than 50 and less than about 2,500 Daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Candidate agents are also found among biomolecules including peptides, saccharides, fatty acids, steroids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Where the screening assay is a binding assay, one or more of the molecules may be joined to a label, where the label can directly or indirectly provide a detectable signal.

Candidate agents may also be found in kinase or phosphatase assays where a kinase or phosphatase substrate such as a protein, a peptide, a lipid, or an organic compound, which may or may not include modifications as further described below, or others are (de)phosphorylated by the described proteins or protein fragments. The kinase or phosphatase can be a described protein (e.g. *Cdk4, fwd,* or *Adk3* homologous kinase or CG7134 or *Pp2C1* homologous phosphatase) or a kinase or phosphatase which is influenced in its activity by a described protein. A therapeutic candidate agent may be identified by its ability to increase or decrease the enzymatic activity of the described proteins. The kinase or phosphatase activity may be detected by change of the chemical, physical or immunological properties of the substrate due to (de)phosphorylation. One example could be the transfer of radioisotopically labelled phosphate groups from an appropriate donor molecule to the kinase substrate, or the cleavage of radioisotopically labelled phosphate groups from a phosphatase substrate, catalyzed by the described polypeptides. The (de)phosphorylation of the substrate may be followed by detection of the substrates autoradiography with techniques well known in the art.

Yet in another example, the change of mass of the substrate due to its (de)phosphorylation may be detected by mass spectrometry techniques. One could also detect the phosphorylation status of a substrate with an analyte discriminating between the phosphorylated and unphosphorylated status of the substrate. Such an analyte may act by having different affinities for the phosphorylated and unphosphorylated forms of the substrate or by having specific affinity for phosphate groups. Such an analyte could be, but is not limited to, an antibody or antibody derivative, a recombinant antibody-like structure, a protein, a nucleic acid, a molecule containing a complexed metal ion, an anion exchange chromatography matrix, an affinity chromatography matrix or any other molecule with phosphorylation dependend selectivity towards the substrate.

Such an analyte could be employed to detect the kinase or phosphatase substrate, which is immobilized on a solid support during or after an enzymatic reaction. If the analyte is an antibody, its binding to the substrate could be detected by a variety of techniques as they are described in Harlow and Lane, 1998, Antibodies, CSH Lab Press, NY. If the analyte molecule is not an antibody, it may be detected by virtue of its chemical, physical or immunological properties, being endogenously associated with it or engineered to it.

Yet in another example the kinase or phosphatase substrate may have features, designed or endogenous, to facilitate its binding or detection in order to generate a signal that is suitable for the analysis of the substrates phosphorylation status. These features may be, but are not limited to, a biotin molecule or derivative thereof, a glutathione-S-transferase moiety, a moiety of six or more consecutive histidine residues, an amino acid sequence or hapten to function as an epitope tag, a fluorochrome, an enzyme or enzyme fragment. The kinase or phosphatase substrate may be linked to these or other features with a molecular spacer arm to avoid steric hindrance..

In one example, the kinase or phosphatase substrate may be labelled with a fluorochrome. The binding of the analyte to the labelled substrate in solution may be followed by the technique of fluorescence polarization as it is described in the literature (see, for example, Deshpande S. et al., (1999) Prog. Biomed. Optics (SPIE) 3603: 261; Parker, G.J. et al. (2000) J. Biomol. Screen. 5: 77-88; Wu P. et al., (1997) Anal. Biochem. 249: 29-36). In a variation of this example, a fluorescent tracer molecule may compete with the substrate for the analyte to detect kinase or phosphatase activity by a technique which is known to those skilled in the art as indirect fluorescence polarization.

In a further example, candidate agents might also be found in kinase assays that utilize bioluminescent measurement of ATP (*fwd* and *Adk3* homologous proteins). The bioluminescent kinase assays are commercially available, e.g. from Bio-Whittaker.

Another technique for drug screening, which may be used, provides for high throughput screening of compounds having suitable binding affinity to the protein of interest as described in published PCT application WO84/03564. In this method, as applied to the described proteins large numbers of different small test compounds, e.g. aptamers, peptides, low-molecular weight compounds etc., are provided or synthesized on a solid substrate, such as plastic pins or some other surface. The test compounds are reacted with a described protein or fragments thereof, and washed. Bound proteins are then detected by methods well known in the art. Purified proteins can also be coated directly onto plates for use in the aforementioned drug screening techniques. Alternatively, non-neutralizing antibodies can be used to capture the peptide and immobilize it on a solid support. In another embodiment, one may use competitive drug screening assays in which neutralising antibodies capable of binding the protein specifically compete with a test compound for binding the *fwd, Pp2C1, Adk3,* CG3860, *Cdk4,* CG7134, or *Eip75B* homologous proteins. In this manner, the antibodies can be used to detect the presence of any peptide, which shares one or more antigenic determinants with the *fwd, Pp2C1, Adk3,* CG3860, *Cdk4,* CG7134, or *Eip75B* homologous proteins.

The nucleic acids encoding the described protein can be used to generate transgenic animals or site-specific gene modifications in cell lines. These transgenic non-human animals are useful in the study of the function and regulation of the described protein in vivo. Transgenic animals, particularly mammalian transgenic animals, can serve as a model system for the investigation of many developmental and cellular processes common to humans. A variety of non-human models of metabolic disorders can be used to test effectors/modulators of the described protein. Misexpression (for example, overexpression or lack of expression) of the described protein, particular feeding conditions, and/or administration of biologically active compounds can create models of metablic disorders.

In one aspect, such assays use mouse models of insulin resistance and/or diabetes, such as mice carrying gene knockouts in the leptin pathway (for example, ob (leptin) or db (leptin receptor) mice). Such mice develop typical symptoms of diabetes, show hepatic lipid accumulation and frequently have increased plasma lipid levels (see Bruning J.C. et al., 1998, supra). Susceptible wild type mice (for example C57BI/6) show similiar symptoms if fed a high fat diet. In addition to testing the expression of the described proteins in such mouse strains (see Examples section), these mice could be used to test whether administration of a candidate effector/modulator alters for example lipid accumulation in the liver, in plasma, or adipose tissues using standard assays well known in the art, such as FPLC, colorimetric assays, blood glucose level tests, insulin tolerance tests and others.

Transgenic animals may be made through homologous recombination in non-human embryonic stem cells, where the normal locus of the gene encoding the described protein is altered. Alternatively, a nucleic acid construct encoding the described protein is injected into oocytes and is randomly integrated into the genome. Vectors for stable integration include plasmids, retroviruses and other animal viruses, yeast artificial chromosomes (YACs), and the like. The modified cells or animals are useful in the study of the function and regulation of the described protein. For example, a series of small deletions and/or substitutions may be made in the gene that encodes the described protein to determine the role of particular domains of the protein, functions in pancreatic differentiation, etc.

Furthermore, variants of the described gene like specific constructs of interest include anti-sense molecules, which will block the expression of the described protein, or expression of dominant negative mutations. A detectable marker, such as for example lac-Z or luciferase may be introduced in the locus of the described gene, where up regulation of expression of the described gene will result in an easily detected change in phenotype.

One may also provide for expression of the described gene or variants thereof in cells or tissues where it is not normally expressed or at abnormal times of development. In addition, by providing expression of the described protein in cells in which they are not normally produced, one can induce changes in cell behavior.

DNA constructs for homologous recombination will comprise at least portions of the described gene with the desired genetic modification, and will include regions of homology to the target locus. DNA constructs for random integration do not need to contain regions of homology to mediate recombination. Conveniently, markers for positive and negative selection are included. DNA constructs for random integration will consist of the nucleic acids encoding the described protein, a regulatory element (promoter), an intron and a poly-adenylation signal. Methods for generating cells having targeted gene modifications through homologous recombination are known in the art. For non-human embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF).

When non-human ES or embryonic cells or somatic pluripotent stem cells have been transfected, they may be used to produce transgenic animals. After transfection, the cells are plated onto a feeder layer in an appropriate medium. Cells containing the construct may be selected by employing a selective medium. After sufficient time for colonies to grow, they are picked and analyzed for the occurrence of homologous recombination or integration of the construct. Those colonies that are positive may then be used for embryo transfection and morula aggregation. Briefly, morulae are obtained from 4 to 6 week old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or in vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the described protein in vivo.

The Figures show:
**Figure 1** shows the content of energy storage triglyceride (TG) of a Drosophila fwd (GadFly Accession Number CG7004) mutant. Shown is the change of triglyceride content of HD-EP(3)30148 flies caused by integration of the P-vector into the annotated transcription unit ('HD-EP30148 (90°C)', column 3) in comparison to controls containing about 2000 fly lines of the proprietary EP collection ('HD-control (90°C)', column 1) and wildtype controls determined in more than 80 independent assays (referred to as 'WT-control (90°C)', column 2).
**Figure 2** shows the molecular organization of the mutated fwd gene locus.
**Figure 3** shows the expression of the fwd homolog in mammalian (mouse) tissues.
**Figure 3A** shows the real-time PCR analysis of the catalytic bets polypeptide of phosphatidylinositol 4 kinase (Pik4cb) expression in wild-type mouse tissues.
**Figure 3B** shows the real-time PCR analysis of Pik4cb expression in different mouse models.
**Figure 3C** shows the real-time PCR analysis of Pik4cb expression in mice fed with a high fat diet compared to mice fed with a control diet..
**Figure 3D** shows the real-time PCR analysis of Pik4cb expression during the differentiation of 3T3-L1 cells from preadipocytes to mature adipocytes.
**Figure 4** shows the expression of the human fwd homolog in mammalian (human) tissue.
**Figure 4A** shows the microarray analysis of phosphatidylinositol 4-kinase, catalytic, beta polypeptide (PIK4CB) expression in human abdominal derived primary adipocyte cells during the differentiation from preadipocytes to mature adipocytes.
**Figure 4B** shows the microarray analysis of PIK4CB expression in a human adipocyte cell line during the differentiation from preadipocytes to mature adipocytes.

The examples illustrate the invention:

### Example 1: Measurement of energy storage metabolites (ESM; triglyceride and/or glycogen) content in Drosophila

Mutant flies are obtained from a fly mutation stock collection. The flies are grown under standard conditions known to those skilled in the art. In the course of the experiment, additional feedings with bakers yeast (Saccharomyces cerevisiae) are provided. The average change of triglyceride and/or glycogen (herein referred to as energy storage metabolites, ESM) content of Drosophila containing the EP-vectors in homozygous viable, hemizygous viable, or homozygous lethal/heterozygous viable integration, was investigated in comparison to control flies grown under the same conditions (see Figures 1, 5, 8, 12, 16, 18, and 20). For determination of triglyceride and glycogen content, flies were incubated for 5 min at 70°C or 90°C in an aqueous buffer using a waterbath, followed by hot extraction. After another 5 min incubation at 70°C or 90°C and mild centrifugation, the triglyceride content of the flies extract was determined using Sigma Triglyceride (INT 336-10 or -20) assay by measuring changes in the optical density according to the manufacturer's protocol. The glycogen content of the flies extract was determined using the Roche Starch UV-method assay (Cat. No. 0207748) by measuring changes in the optical density according to the manufacturer's protocol. As a reference the protein content of the same extract was measured using BIO-RAD DC Protein Assay according to the manufacturer's protocol. These experiments and assays were repeated several times.

The average triglyceride level (µg triglyceride/µg protein) of all flies of the EP collection (referred to as 'EP-control') is shown as 100% in the first columns in Figures 16 and 20. The average triglyceride level (µg triglyceride/µg protein) of 2108 flies of the proprietary EP collection (referred to as ¹HD-control (90°C)') is shown as 100% in the first columns in Figures 1, 8, 12, and 18. The average triglyceride level (µg triglyceride/µg protein) of Drosophila wildtype strain Oregon R flies determined in 84 independent assays (referred to as "WT-control (90°C)') is shown as 102% in the second columns in Figure 1, 8, 12, and 18.

The average triglyceride level (µg triglyceride/µg protein) of 883 fly lines of the proprietary EP-collection determined at 70°C (referred to as 'HD-control (70°C)') is shown as 100% in the fourth column in Figure 8. The average triglyceride level of Drosophila wildtype strain Oregon R flies determined in 4 independent assays at 70°C (referred to as 'WT-control (70°C)') is shown as 116% in the fifth column in Figure 8. The average glycogen level (µg glycogen/µg protein) of 19 fly lines of the proprietary EP-collection with X-chromosomal insertions, determined at 70°C (referred to as 'HD-control (70°C)') is shown as 100% in the first column in Figure 5. The average glycogen level (µg glycogen/µg protein) of 20 fly lines of the proprietary EP-collection with X-chromosomal insertions, determined at 90°C (referred to as 'HD-control (90°C)') is shown as 100% in the third column in Figure 5. Standard deviations of the measurements are shown as thin bars.

HD-EP(3)30148 homozygous flies ('HD-EP30148 (90°C)', column 3 in Figure 1), HD-EP(3)36627 heterozygous flies ('HD-36627/TM3 (90°C)', column 3, and 'HD-36627/TM3 (70°C)', column 6 in Figure 8), HD-EP(2)25831 homozygous flies ('HD-EP25831 (90°C)', column 3 in Figure 12), HD-EP(2)21120 homozygous flies ('HD-EP21120' column 2 in Figure 16), HD-EP(2)20271 homozygous flies ('HD-20271 (TG)', column 3 in Figure 18), and HD-EP(3)30293 heterozygous flies ('HD-EP30293 / TM3', column 2 in Figure 20) show constantly a higher triglyceride content than the controls. HD-EP(X)10310 hemizygous flies show constantly a slightly higher triglyceride content than the controls (data not shown). HD-EP(X)10310 hemizygous flies ('HD-10310 (70°C)', column 2 in Figure5, and 'HD-10310 (90°C)', column 4 in Figure 5) show constantly a higher glycogen content than the controls, determined at different temperatures. Therefore, the loss of gene activity is responsible for changes in the metabolism of the energy storage metabolites.

### Example 2: Identification of Drosophila genes for changes in triglyceride and/or glycogen levels

Nucleic acids encoding the described proteins were identified using a plasmid-rescue technique. Genomic DNA sequences were isolated that are localized adjacent to the EP vectors (herein HD-EP(3)30148, HD-EP(X)10310, HD-EP(3)36627, HD-EP(2)25831, HD-EP(2)21120, HD-EP(2)20271, or HD-EP(3)30293) integration. Using those isolated genomic sequences public databases like Berkeley Drosophila Genome Project (GadFly) were screened, thereby identifying the integration sites of the vectors, and the corresponding genes. The molecular organization of these gene loci is shown in Figure 2.

In Figure 2 genomic DNA sequence is represented by the assembly as a horizontal black scaled double-headed arrow that includes the integration sites of the vectors for line HD-EP(3)30148. Ticks represent the length in basepairs of the genomic DNA (1000 base pairs (Figure 2 per tick). The part of the figure above the double-headed arrow represents the sense strand, the part below the arrow represents the antisense strand. The grey arrows in the upper part of the figures represent BAC clones, the black arrows in the topmost part of the figures represent the sections of the chromosomes. The insertion sites of the P-elements in the Drosophila lines are shown as triangles and are labeled. The cDNA sequences of the predicted genes (as predicted by the Berkeley Drosophila Genome Project, GadFly and by Magpie) are shown as dark grey bars (exons), linked by dark grey lines (introns), and are labeled (see also key at the bottom of the figures).

The HD-EP(3)30148 vector is homozygous viable integrated 122 base pairs 5' of transcription variant CG7004-RA of the Drosophila *fwd* gene in antisense orientation. The chromosomal localization site of integration of the vector of HD-EP(3)30148 is at gene locus 3L, 61C1. In Figure 2, the transcript variants of the cDNA of the Drosophila gene *fwd* are shown in the lower half of the figure, and are labeled. The integation site of HD-EP(3)30148 is indicated with a black triangle 5' of the first exon of the predicted transcript variants of the *fwd gene.*

Therefore, expression of the cDNAs encoding the described proteins could be affected by integration of the vectors, leading to a change in the amount of energy storage triglycerides and/or glycogen.

### Example 3: Identification of human homologous genes and proteins

The Drosophila genes and proteins encoded thereby with functions in the regulation of triglyceride and/or glycogen metabolism were further analysed using the BLAST algorithm searching in publicly available sequence databases and mammalian homologs were identified (see Table 1).

The term "polynucleotide comprising the nucleotide sequence as shown in GenBank Accession number" relates to the expressible gene of the nucleotide sequences deposited under the corresponding GenBank Accession number. The term "GenBank Accession number" relates to NCBI GenBank database entries (Ref.: Benson et al., (2000) Nucleic Acids Res. 28: 15-18). The term "IPI Accession Number" relates to ENSEMBL International Protein Index entries (http://www.ensembl.org/IPI/; Hubbard T. et al., (2002) Nucleic Acids Research 30: 38-41). Sequences homologous to Drosphila *fwd, Pp2C1, Adk3,* CG3860, *Cdk4,* CG7134, or *Eip75B* were identified using the publicly available program BLASTP 2.2.3 of the non-redundant protein data base of the National Center for Biotechnology Information (NCBI) (see, Altschul S.F. et al., (1997) Nucleic Acids Res. 25: 3389-3402).

**Table 1: Human homologs of the Drosophila (Dm) genes**

| **Dm gene** | **Homo sapiens homologous genes and proteins** | | |
|---|---|---|---|
| **Acc. No. *Name*** | **Accession Number** | | **Name** |
| | **cDNA** | **Protein** | |
| CG7004 *fwd* | NM_002651 | NP_002642 | phosphatidylinositol 4-kinase, catalytic, beta polypeptide (PIK4CB) |
| CG2984 *Pp2C1* | NM_003620 | NP_003611 | protein phosphatase 1D, magnesium-dependent, delta isoform (PPM1D) |
| CG6612 *Adk3* | NM 016282 NM 013410 | NP 057366 NP 037542 | adenylate kinase 3 like 1 adenylate kinase 3 (AK3) |
| *CG3860* | NM_018030 | NP_060500 | oxysterol binding protein-like 1A (OSBPL1A), transcript variant OSBPL 1 A; isoform A |
| | NM_080597 | NP_542164 | oxysterol-binding protein-like 1A (OSBPL1A), transcript variant OSBPL1B; isoform B |
| | NM_133268 | NP_579802 | oxysterol-binding protein-like 1A (OSBPL1A), transcript variant OSBPL1C; isoform C |
| | NM_014835 | NP_055650 | oxysterol binding protein-like protein 2 (OSBPL2), transcript variant 1; isoform 1 |
| | NM_144498 | NP_653081 | oxysterol-binding protein-like protein 2 (OSBPL2), transcript variant 2; isoform 2 |
| CG5072 | NM_000075 | NP_000066 | cyclin-dependent kinase 4 (CDK4), transcript |
| *Cdk4* | | | variant 1; isoform 1 |
| | NM_052984 | NP_443710 | cyclin-dependent kinase 4 (CDK4), transcript variant 2; isoform 2 |
| | NM 001259 | NP 001250 | cyclin-dependent kinase 6 (CDK6) |
| CG7134 | NM_003672 | NP_003663 | CDC 14 cell division cycle 14 homolog A (S. cerevisiae) (CDC 14A), transcript variant 1; isoform 1 |
| | NM_033312 | NP_201569 | CDC14 cell division cycle 14 homolog A (S. cerevisiae) (CDC14A), transcript variant 2; isoform 2 |
| | NM_033313 | NP_201570 | CDC14 cell division cycle 14 homolog A (S. cerevisiae) (CDC14A), transcript variant 3; isoform 3 |
| | NM_003671 | NP_003662 | CDC14 cell division cycle 14 homolog B (S. cerevisiae) (CDC 14B), transcript variant 1; isoform 1 |
| | NM_033331 | NP_201588 | CDC14 cell division cycle 14 homolog B (S. cerevisiae) (CDC14B), transcript variant 2; isoform 2 |
| | NM_033332 | NP_201589 | CDC14 cell division cycle 14 homolog B (S. cerevisiae) (CDC14B), transcript variant 3; isoform 3 |
| CG8127 *Eip75B* | NM_021724 | NP_068370 | nuclear receptor subfamily 1, group D, member 1 (NR1D1) |
| | NM_005126 | NP_005117 | nuclear receptor subfamily 1, group D, member 2 (NR1D2) |

*fwd, Pp2C1, Adk3,* CG3860, *Cdk4,* CG7134, or *Eip75B* homologous proteins and nucleic acid molecules coding therefore are obtainable from insect or vertebrate species, e.g. mammals or birds. Also disclosed are nucleic acids as described in Table 1.

The mouse homologous cDNAs encoding the described polypeptides were identified as GenBank Accession Numbers XM_196305 and XM_205921 (for the mouse homologs of PIK4CB), GenBank Accession Number NM_016910 (for the mouse homolog of PPM1D), XM_129200 (for the mouse homolog of AK3L1), AB020239 and D85036 (for the mouse homolog of AK3), NM_020573 (for the mouse homolog of OSBPL1A), NM_144500 (for the mouse homolog of OSBPL2), NM_009870 (for the mouse homolog of CDK4), NM_009873 (for the mouse homolog of CDK6), IPI Accession Numbers IPI00134094 and IPI00111965 (for the mouse homologs of CDC14A), IPI Accession Numbers IPI00126617, IPI00126961, and IPI00124775 (for the mouse homologs of CDC14B), NM_145434 and XM_126627 (for the mouse homologs of NR1D1), and NM_011584 (for the mouse homologs of NR1D2).

### Example 5: Expression of the polypeptides in mammalian tissues

### Example 5A: Expression of the polypeptides in mouse tissues

To analyse the expression of the described polypeptides in mammalian tissues, several mouse strains (preferably mice strains C57BI/6J, C57BI/6 ob/ob and C57BI/KS db/db which are standard model systems in obesity and diabetes research) were purchased from Harlan Winkelmann (33178 Borchen, Germany) and maintained under constant temperature (preferably 22°C), 40 per cent humidity and a light / dark cycle of preferably 14 / 10 hours. The mice were fed a standard chow (for example, from ssniff Spezialitäten GmbH, order number ssniff M-Z V1126-000). For the fasting experiment ("fasted wild type mice"), wild type mice were starved for 48 h without food, but only water supplied ad libitum (see, for example, Schnetzler B. et al., (1993) J Clin Invest 92: 272-280, Mizuno T.M. et al., (1996) Proc Natl Acad Sci USA 93: 3434-3438). In a further experiment wild-type (wt) mice were fed a control diet (preferably Altromin C1057 mod control, 4.5% crude fat) or high fat diet (preferably Altromin C1057mod. high fat, 23.5% crude fat). Animals were sacrificed at an age of 6 to 8 weeks. The animal tissues were isolated according to standard procedures known to those skilled in the art, snap frozen in liquid nitrogen and stored at -80°C until needed.

For analyzing the role of the described proteins in the *in vitro* differentiation of mammalian cell culture cells for the conversion of pre-adipocytes to adipocytes, mammalian fibroblast (3T3-L1) cells (e.g., Green H. and Kehinde O., (1974) Cell 1: 113-116) were obtained from the American Tissue Culture Collection (ATCC, Hanassas, VA, USA; ATCC- CL 173). 3T3-L1 cells were maintained as fibroblasts and differentiated into adipocytes as described in the prior art (e.g., Qiu Z. et al., (2001) J. Biol. Chem. 276: 11988-11995; Slieker L.J. et al., (1998) BBRC 251: 225-229). In brief, cells were plated in DMEM/10% FCS (Invitrogen, Karlsruhe, Germany) at 50,000 cells/well in duplicates in 6-well plastic dishes and cultured in a humidified atmosphere of 5% CO₂ at 37°C. At confluence (defined as day 0: d0) cells were transferred to serum-free (SF) medium, containing DMEM/HamF12 (3:1; Invitrogen), fetuin (300 µg/ml; Sigma, Munich, Germany), transferrin (2 µg/ml; Sigma), pantothenate (17µM; Sigma), biotin (1µM; Sigma), and EGF (0.8nM; Hoffmann-La Roche, Basel, Switzerland). Differentiation was induced by adding dexamethasone (DEX; 1µM; Sigma), 3-methyl-isobutyl-1-methylxanthine (MIX; 0.5mM; Sigma), and bovine insulin (5µg/ml; Invitrogen). Four days after confluence (d4), cells were kept in SF medium, containing bovine insulin (5µ/ml) until differentiation was completed. At various time points of the differentiation procedure, beginning with day 0 (day of confluence) and day 2 (hormone addition; for example, dexamethasone and 3-isobutyl-1'-methylxanthine), up to 12 days of differentiation, suitable aliquots of cells were taken every two days.

RNA was isolated from mouse tissues or cell culture cells using Trizol Reagent (for example, from Invitrogen, Karisruhe, Germany) and further purified with the RNeasy Kit (for example, from Qiagen, Germany) in combination with a DNase-treatment according to the instructions of the manufacturers and as known to those skilled in the art. Total RNA was reverse transcribed (preferably using Superscript II RNaseH⁻ Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

Taqman analysis was performed preferably using the following primer/probe pairs:

For the amplification of mouse phosphatidylinositol 4-kinase, catalytic, beta polypeptide (Pik4cb) sequence (GenBank Accession Number XM_196305):
Mouse Pik4cb forward primer (SEQ ID NO: 1): 5'- TGG AAG CAA GGA AGC TGA GAA C -3'; mouse Pik4cb reverse primer (SEQ ID NO: 2): 5'- GCT CGG AAG CAC ATG GAC A -3'; Pik4cb Taqman probe (SEQ ID NO: 3): (5/6-FAM)- TGC TGC ATG ATC TCC ACA ATC TGT ACC A -(5/6-TAMRA).

For the amplification of mouse protein phosphatase 1D magnesium-dependent, delta isoform (Ppm1d) sequence (GenBank Accession Number NM_016910):
Mouse Ppm1d forward primer (SEQ ID NO: 4): 5'- ATG GCC AAA GAC TAT GAC AGG TC -3'; mouse Ppm1d reverse primer (SEQ ID NO: 5): 5'- TGC CCC GTA TTA TAA CCA CAC TG -3'; Ppm1d Taqman probe (SEQ ID NO: 6): (5/6-FAM)- TCC CAG CAC GTC CGG GAC AAC T -(516-TAMRA).

For the amplification of mouse adenylate kinase 3 alpha like (AkI3I) sequence (GenBank Accession Number XM_129200):
Mouse AkI3I forward primer (SEQ ID NO:7): 5'- CAG TGG TCG AGT ATA CAA CAT TGA ATT -3'; mouse AkI3I reverse primer (SEQ ID NO: 8): 5'-GCT GAA TCA GAG GTT CTC CGG -3'; AkI3I Taqman probe (SEQ ID NO: 9): (5/6-FAM)- CCA AGA CTG TGG GCA TTG ATG ACC TG -(5/6-TAMRA). For the amplification of mouse adenylate kinase 4 (Ak4) sequence (GenBank

Accession Number D85036):
Mouse Ak4 forward primer (SEQ ID NO:10): 5'- CGC CCA GAA CTT TG GCC -3'; mouse Ak4 reverse primer (SEQ ID NO: 11): 5'- CGG TCT TGA GGT TCT CCC G -3'; Ak4 Taqman probe (SEQ ID NO: 12): (5/6-FAM)-CCA GCA TCT CTC CAG CGG CCA CT -(5/6-TAMRA).

For the amplification of mouse oxysterol binding protein-like 1A (Osbpl1a) sequence (GenBank Accession Number NM_020573):
Mouse Osbpl1a forward primer (SEQ ID NO: 13): 5'- TGG CTG CAT TTG CTG TGT CT -3'; mouse Osbpl1a reverse primer (SEQ ID NO: 14): 5'- CGG GTT GAA GGG CTT TCC -3'; Osbpl1a Taqman probe (SEQ ID NO: 15): (5/6-FAM)- CGC CTC TCA GTG GGA GCG CAC -(5/6-TAMRA).

For the amplification of mouse oxysterol binding protein-like 2 (Osbpl2) sequence (GenBank Accession Number NM_144500):
Mouse Osbpl2 forward primer (SEQ ID NO: 16): 5'- CAT GTG TAC CTC ATT CAC AAA GCC -3'; mouse Osbpl2 reverse primer (SEQ ID NO: 17): 5'-GGC AGC GAC AGA CTG CAT T -3'; Osbpl2 Taqman probe (SEQ ID NO: 18): (5/6-FAM)- CAA GTC AGT CCC AGC CGC TGG AG -(5/6-TAMRA).

For the amplification of mouse nuclear receptor subfamily 1, group D, member 1 (Nr1d1) sequence (GenBank Accession Number NM_145434):
Mouse Nr1d1 forward primer (SEQ ID NO: 19): 5'- CGG CTC AGC GTC ATA ATG AAG -3'; mouse Nr1d1 reverse primer (SEQ ID NO: 20): 5'- AGG CCA GGT AGG CGG GTA -3'; Nr1d1 Taqman probe (SEQ ID NO: 21): (5/6-FAM)- CTG AAT GGT CTA CGC CAG GGC CC -(5/6-TAMRA).

For the amplification of mouse nuclear receptor subfamily 1, group D, member 2 (Nr1d2) sequence (GenBank Accession Number NM_011584):
Mouse Nr1d2 forward primer (SEQ ID NO: 22): 5'- ACT GTG ATG CCA ACG GCA -3'; mouse Nr1d2 reverse primer (SEQ ID NO: 23): 5'- CTG TGC GGT CAC TCT TCA GAA C -3'; Nr1d2 Taqman probe (SEQ ID NO: 24): (5/6-FAM)- TCC CAA GAA CGC TGA TAT CTC TAG CAT CGA -(5/6-TAMRA).

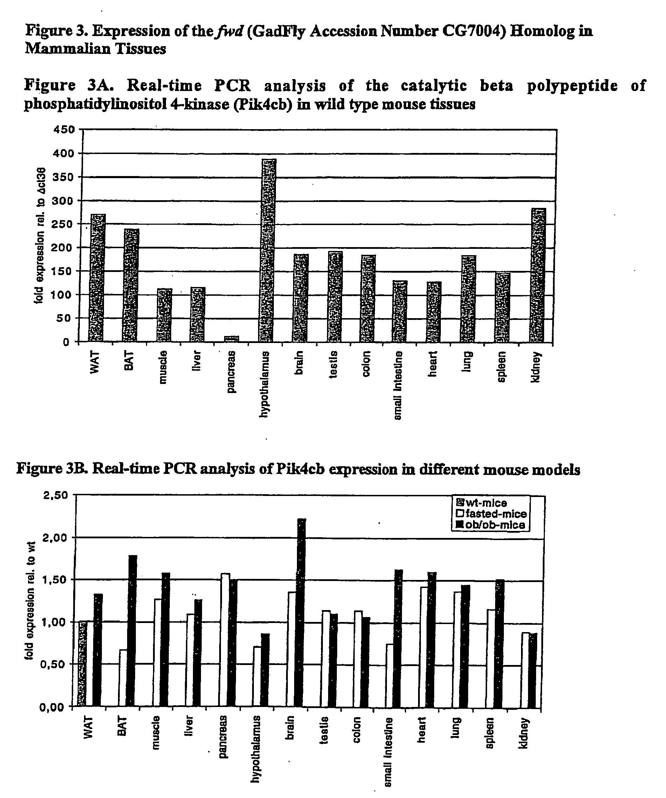

In the figures the relative RNA-expression is shown on the Y-axis. In Figures 3A-C, 7A-C, 10A-B, 10D-E, 14A-B, 14D-E, 22A-B, and 22D-E, the tissues tested are given on the X-axis. "WAT" refers to white adipose tissue, "BAT" refers to brown adipose tissue.

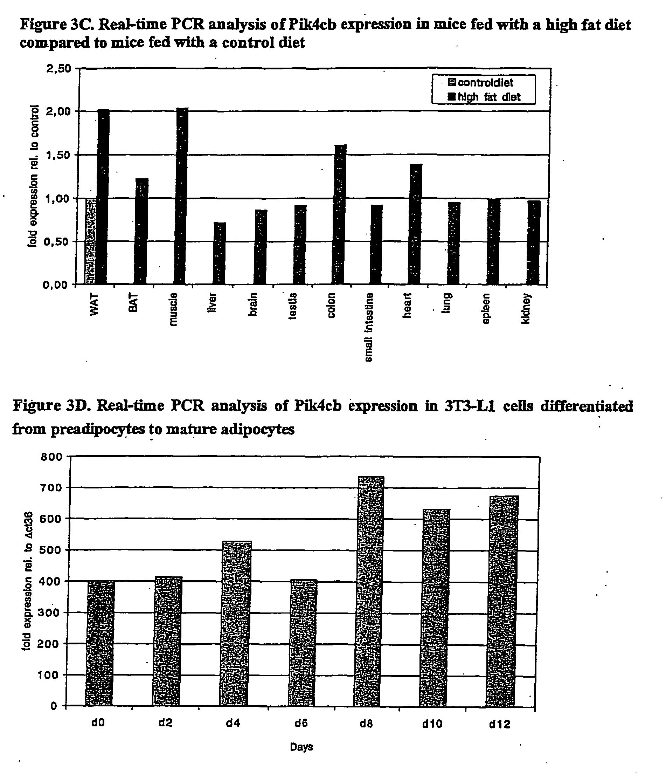

In Figures 10A-B, 10D-E, 14A-B, and 14D-E, the panel of the wild type mice tissues comprises WAT, BAT, muscle, liver, pancreas, hypothalamus, brain, testis, colon, small intestine, heart, lung, spleen, and kidney, and the panel of the control diet-mice tissues comprises WAT, BAT, muscle, liver, brain, testis, colon, small intestine, heart, lung, spleen, and kidney. In Figures 3D, 7D, 10C, 10F, 14C, 14F, 22C, and 22F, the X-axis represents the time axis. "d0" refers to day 0 (start of the experiment), "d2" - "d12"refers to day 2 - day 12 of adipocyte differentiation.

The function of the described proteins in metabolism was further validated by analyzing the expression of the transcripts in different tissues and by analyzing the role in adipocyte differentiation.

Mouse models of insulin resistance and/or diabetes were used, such as mice carrying gene knockouts in the leptin pathway (for example, *ob*/*ob* (leptin) or *db*/*db* (leptin receptor/ligand) mice) to study the expression of the described proteins. Such mice develop typical symptoms of diabetes, show hepatic lipid accumulation and frequently have increased plasma lipid levels (see Bruning J.C. et al, (1998) Mol. Cell. 2: 559-569).

Further expression of the mRNAs encoding the described proteins was also examined in susceptible wild type mice (for example, C57BI/6) that show symptoms of diabetes, lipid accumulation, and high plasma lipid levels, if fed a high fat diet.

Expression profiling studies confirm the particular relevance of the described proteins as regulators of energy metabolism in mammals.

Taqman analysis revealed that the catalytic betya polypeptide of phosphatidylinositol 4-kinase (Pik4cb) is expressed in several mammalian tissues, showing highest level of expression in hypothalamus and higher levels in further tissues, e.g. WAT, BAT, muscle, liver, brain, testis, colon, small intestine, heart, lung, spleen, and kidney. Furthermore Pik4cb is expressed on a lower but still robust levels in the pancreas of wild type mice as depicted in Figure 3A. We found, for example, that the expression of Pik4cb is up-regulated in the brain of ob/ob mice compared to wildtype mice (see Figure 3B). In wild type mice fed a high fat diet, the expression of Pik4cb is up-regulated in WAT and muscle, as depicted in Figure 3C. We further show in this invention (see Figure 3D) that the Pik4cb mRNA is expressed and slightly up-regulated during the differentiation into mature adipocyctes. The regulated expression of Pik4cb in different animal models used to study metabolic disorders, together with the regulation during the differentiation from preadipocytes to mature adipocytes, suggest that it plays an essential role in cellular metabolism.

### Example 6. Analysis of the differential expression of transcripts of the described proteins in human tissues

RNA preparation from human primary adipose tissues was done as described in Example 5. The target preparation, hybridization, and scanning was performed as described in the manufactures manual (see Affymetrix Technical Manual, 2002, obtained from Affmetrix, Santa Clara, USA).

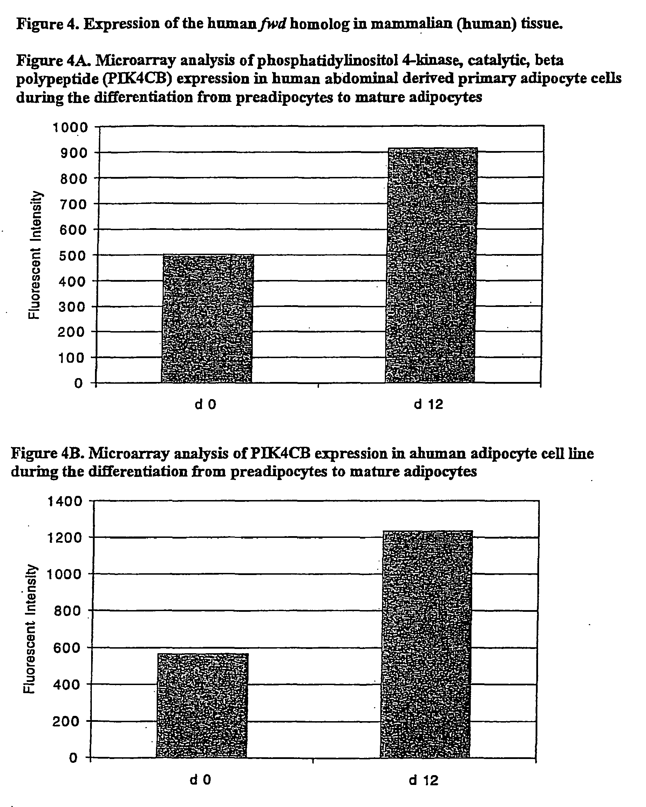

In Figures 4, 11, and 15 the X-axis represents the time axis, shown are day 0 and day 12 of adipocyte differentiation. The Y-axis represents the flourescent intensity. The expression analysis (using Affymetrix GeneChips) of the phosphatidylinositol 4-kinase, catalytic, beta polypeptide (PIK4CB), adenylate kinase 3 like 1 (AK3L1), adenylate kinase 3 (AK3), oxysterol binding protein-like 1A (OSBPL1A), and oxysterol binding protein-like 2 (OSBPL2) genes using human abdominal derived primary adipocytes and human adipocyte cell line (SGBS) differentiation, clearly shows differential expression of human PIK4CB, AK3L1, AK3, OSBPL1A, and OSBPL2 genes in adipocytes. Several independent experiments were done. The experiments further show that the PIK4CB, AK3L1, AK3, and OSBPL1A, and transcripts are most abundant at day 12 compared to day 0 during differentiation (see Figures 4, 11, and 15A-B). The experiments further show that the OSBPL2 transcript is most abundant at day 0 compared to day 12 during differentiation (see Figure 15C).

Thus, the PIK4CB, AK3L1, AK3, and OSBPL1A proteins have to be significantly increased in order for the preadipocyctes to differentiate into mature adipocycte, and the OSBPL2 protein has to be significantly decreased in order for the preadipocyctes to differentiate into mature adipocycte. Therefore, PIK4CB, AK3L1, AK3, and OSBPL1A in preadipocyctes have the potential to enhance adipose differentiation, and OSBPL2 in preadipocyctes has the potential to inhibit adipose differentiation.

Therefore, PIK4CB, AK3L1, AK3, OSBPL1A, and OSBPL2 proteins might play an essential role in the regulation of human metabolism, in particular in the regulation of adipogenesis and thus it might play an essential role in obesity, diabetes, and/or metabolic syndrome.

### Example 7: Generation and analysis of transgenic mice

Mouse cDNA is isolated from mouse brown adipose tissue (BAT) using standard protocols as known to those skilled in the art. The cDNA is amplified by RT-PCR and point mutations are introduced into the cDNA.

The resulting mutated cDNA is cloned into a suitable transgenic expression vector. The transgene is microinjected into the male pronucleus of fertilized mouse embryos (preferably strain C57/BL6/CBA F1 (Harlan Winkelmann). Injected embryos are transferred into pseudo-pregnant foster mice. Transgenic founders are detected by PCR analysis. Two independent transgenic mouse lines containing the construct are established and kept on a C57/BL6 background. Briefly, founder animals are backcrossed with C57/BL6 mice to generate F1 mice for analysis: Transgenic mice are continously bred onto the C57/BI6 background. The expression of the described proteins can be analyzed by taqman analysis as described above, and further analysis of the mice can be done as known to those skilled in the art.

## Claims

1. A method of identifying a (poly)peptide involved in the regulation of energy homeostasis or/and metabolism of triglycerides in a mammal comprising the steps of
(a) contacting a collection of (poly)peptides with a fwd homologous polypeptide being PIK4CB (NP_002642) under conditions that allow binding of said (poly)peptides;
(b) removing (poly)peptides which do not bind and
(c) identifying (poly)peptides that bind to said PIK4CB (NP_002642).

2. A method for diagnosing a disease selected from metabolic syndrome or obesity comprising the steps of
(i) assessing the presence or amount of PIK4CB protein (NP_002642) or PIK4CB DNA (NM_002651) in a sample from a patient in vitro,
(ii) comparing the result of step (i) with a standard value obtained from a normal subject,
wherein a deviation between the standard value and the value obtained from the patient establishes the parameters for diagnosing the disease.

3. The diagnostic method of claim 2, wherein the sample is derived from body fluids, cell extracts or biopsied tissues.

4. The diagnostic method of claim 2 or 3, wherein the assessment of the presence or amount of PIK4CB protein (NP_002642) comprises combining the sample with antibodies to the PIK4CB protein under conditions suitable for complex formation.

5. The diagnostic method of claim 2 or 3, wherein the assessment of the presence or amount of PIK4CB DNA (NM_002651) comprises combining the sample with hybridization probes for PIK4CB DNA.

6. The diagnostic method of any one of claims 2-5 for monitoring the progress of patients receiving treatment for metabolic syndrome or obesity.

## Patentansprüche

1. Verfahren zur Identifizierung eines (Poly)peptids, dass an der Regulation von Energiehomöostase oder/und dem Stoffwechsel von Triglyceriden in einem Säugetier beteiligt ist, umfassend die Schritte
(a) Kontaktieren einer Kollektion von (Poly)peptiden mit einem *fwd* homologen Polypeptid, dem PIK4CB (NP_002642) unter Bedingungen welche die Bindung der (Poly)peptide erlaubt;
(b) Entfernen der (Poly)peptide welche nicht binden und
(c) Identifizieren der (Poly)peptide welche an das PIK4CB (NP_002642) binden.

2. Verfahren zur Diagnose einer Krankheit, ausgewählt aus metabolischem Syndrom oder Obesitas umfassend die Schritte
(i) Beurteilen der Gegenwart oder Menge von PIK4CB Protein (NP_002642) oder PIK4CB DNA (NM_002651) in einer Probe aus einem Patienten in vitro,
(ii) Vergleichen des Ergebnisses aus Schritt (i) mit einem Standardwert der aus einem normalen Individuum erhalten wurde,
worin eine Abweichung zwischen dem Standardwert und dem Wert, welcher von dem Patienten erhalten wurde, die Parameter zur Diagnose der Krankheit feststellt.

3. Diagnostikverfahren nach Anspruch 2, worin die Probe aus Körperflüssigkeiten, Zellextrakten oder biopsierten Geweben stammt.

4. Diagnostikverfahren nach Anspruch 2 oder 3, worin die Beurteilung der Gegenwart oder Menge von PIK4CB Protein (NP_002642), die Kombination der Probe mit Antikörpern gegen das PIK4CB Protein unter Bedingungen, die für Komplexbildung geeignet sind, umfasst.

5. Diagnostikverfahren nach Anspruch 2 oder 3, worin die Beurteilung der Gegenwart oder Menge von PIK4CB DNA (NM_002651) die Kombination der Probe mit Hybridisierungssonde für PIK4CB DNA umfasst.

6. Diagnostikverfahren nach einem der Ansprüche 2-5 zur Überwachung des Verlaufs von Patienten welche eine Behandlung für metabolisches Syndrom oder Obesitas erhalten.

## Revendications

1. Procédé d'identification d'un (poly)peptide impliqué dans la régulation de l'homéostase énergétique et/ou le métabolisme des triglycérides chez un mammifère comprenant les étapes consistant à :
(a) mettre en contact une collection de (poly)peptides avec un polypeptide homologue *fwd* qui est PIK4CB (NP_002642) dans des conditions qui permettent la liaison desdits (poly)peptides ;
(b) éliminer les (poly)peptides qui ne se lient pas et
(c) identifier les (poly)peptides qui se lient à ladite PIK4CB (NP_002642).

2. Procédé pour diagnostiquer une maladie choisie parmi un syndrome métabolique ou une obésité comprenant les étapes consistant à :
(i) évaluer la présence ou la quantité de protéine PIK4CB (NP_002642) ou d'ADN de PIK4CB (NM_002651) dans un échantillon provenant d'un patient *in vitro,*
(ii) comparer le résultat de l'étape (i) avec une valeur standard obtenue à partir d'un sujet normal,
dans lequel un écart entre la valeur standard et la valeur obtenue à partir du patient établit les paramètres pour diagnostiquer la maladie.

3. Procédé de diagnostic selon la revendication 2, dans lequel l'échantillon est dérivé de fluides corporels, d'extraits cellulaires ou de tissus biopsiés.

4. Procédé de diagnostic selon la revendication 2 ou 3, dans lequel l'évaluation de la présence ou de la quantité de protéine PIK4CB (NP_002642) comprend la combinaison de l'échantillon avec des anticorps contre la protéine PIK4CB dans des conditions appropriées pour la formation d'un complexe.

5. Procédé de diagnostic selon la revendication 2 ou 3, dans lequel l'évaluation de la présence ou de la quantité d'ADN de PIK4CB (NM_002651) comprend la combinaison de l'échantillon avec des sondes d'hybridation pour l'ADN de PIK4CB.

6. Procédé de diagnostic selon l'une quelconque des revendications 2 à 5, destiné à la surveillance des progrès des patients recevant un traitement pour un syndrome métabolique ou une obésité.
